# DEMANDE DE BREVET EUROPEEN

(11) **EP 1 437 125 A1**
(43) Date de publication de la demande: **14.07.2004**
(21) Numéro de dépôt: 03293010.9
(22) Date de dépôt: 02.12.2003
(51) Int. Cl.: A61K 7/48

(54) **Composition sous forme d'émulsion E/H contenant des cires, et son utilisation dans le domaine cosmétique**

(30) Priorité: 03.01.2003 FR 0300042
(71) Demandeur: L'OREAL, 75008 Paris (FR)
(72) Inventeur: Chevalier, Véronique, 94440 Villecresnes (FR)
(74) Mandataire: Rasson, Catherine

(57) **Abrégé**

L'invention se rapporte à une composition constituée d'une émulsion E/H comprenant une phase aqueuse dispersée dans une phase huileuse, la phase huileuse contenant au moins une cire et se présentant sous forme d'une pâte souple à température ambiante, caractérisée en ce qu'elle contient au moins un polymère amphiphile choisi parmi les oligomères ou polymères dérivés de polyoléfine.

La composition se présente sous forme d'un produit souple, notamment sous forme de crème.

La présente invention se rapporte aussi aux utilisations de ladite composition dans les domaines cosmétique et dermatologique, notamment pour le soin, le traitement et/ou le maquillage de la peau et/ou des muqueuses.

L'invention concerne également un procédé de préparation de cette composition, caractérisé en ce que l'on effectue au moins une étape de cette préparation à l'aide d'un mélangeur-extrudeur.

## Description

L'invention se rapporte à une composition se présentant sous forme d'une émulsion E/H contenant des cires et un polymère amphiphile, et à l'utilisation de la dite composition, en particulier pour le soin, le traitement et/ou le maquillage de la peau du corps ou du visage, des cils et/ou des lèvres, et/ou pour le soin des cheveux.

L'invention concerne également un procédé de préparation de cette composition selon lequel on effectue au moins une étape de cette préparation à l'aide d'un mélangeur-extrudeur.

Il est connu par les documents EP-A-1,005,856 et EP-A-1,005,857,de préparer des compositions cosmétiques sous forme d'émulsions E/H contenant un taux élevé de cires, c'est-à-dire au moins 5 % de cires, et se présentant sous forme de produits souples tels qu'une crème. Ces émulsions ont l'avantage d'être plus fraîches lors de l'application sur la peau que les compositions anhydres cireuses. Elles ont aussi pour particularité que la phase huileuse de l'émulsion se présente à température ambiante (c'est-à-dire environ 25°C), sous forme d'une pâte souple avant l'incorporation de la phase aqueuse pour constituer l'émulsion E/H, ce qui présente l'avantage de pouvoir incorporer une grande quantité de cire tout en obtenant une émulsion stable. De plus, c'est particulièrement avantageux lorsque l'on souhaite introduire des composés thermosensibles du fait que le mélange des phases aqueuse et huileuse est fait à froid. Toutefois, les émulsions décrites dans ces documents contiennent un émulsionnant siliconé. Or, la présence d'émulsionnants peut être préjudiciable lors de l'utilisation de ces compositions par les sujets à peau sensible, et on cherche de plus en plus à obtenir des émulsions exemptes de tensioactifs émulsionnants, et donc mieux tolérées.

Il subsiste donc le besoin d'émulsions E/H contenant des cires et constituant des crèmes plus ou moins fluides, qui soient stables tout en présentant de bonnes propriétés cosmétiques et tout en étant adaptées aux peaux sensibles.

La demanderesse a découvert de façon inattendue que l'utilisation de polymères amphiphiles dérivés de polyoléfines permettait de résoudre le problème à la base de l'invention et d'avoir une composition sous forme de crème, à la fois stable, souple, douce et bien tolérée par tout type de peaux.

Certes, il est connu par le document EP-A-1,172,089 de préparer des émulsions E/H contenant des polymères dérivés de polyoléfines. Toutefois, les émulsions décrites dans ce document ne contiennent pas de cires dont la présence est un facteur de déstabilisation de l'émulsion, et l'utilisation de cires est même déconseillée dans ce document (voir col. 1, lignes 55 à 58). Par ailleurs, d'autres polymères émulsionnants sont connus pour remplacer les tensioactifs dans les émulsions E/H, comme par exemple les polymères anioniques d'acide isophtalique ou d'acide sulfoisophtalique, décrits dans le document EP-A-864320, mais ces polymères ne permettent pas d'obtenir des émulsions E/H stables quand ces émulsions contiennent une grande quantité de cires solides, c'est-à-dire de cires ayant une température de fusion commençante supérieure ou égale à 50°C. Ainsi, il existe différentes catégories chimiques de polymères émulsionnants, mais seuls les polymères utilisés selon l'invention permettent d'obtenir une composition stable et ayant de bonnes qualités cosmétiques tout en ayant un taux élevé de cire.

L'invention a pour objet une composition constituée d'une émulsion E/H comprenant, dans un milieu physiologiquement acceptable, une phase aqueuse dispersée dans une phase huileuse, la phase huileuse contenant au moins une cire et se présentant sous forme d'une pâte souple à température ambiante, caractérisée en ce qu'elle contient au moins un polymère amphiphile choisi parmi les oligomères ou polymères dérivés de polyoléfine, comportant une partie apolaire polyoléfinique comprenant au moins 40 atomes de carbone et au moins une partie polaire.

Selon un mode préféré de réalisation de l'invention, la pâte souple de la phase huileuse de la composition selon l'invention est préparée au moins partiellement à l'aide d'un mélangeur-extrudeur.

On entend par "pâte souple", une pâte dont on peut mesurer la viscosité, par opposition à la structure solide d'un bâton ou stick, dont on ne peut pas mesurer la viscosité. La viscosité dynamique de la pâte souple à 25°C est généralement comprise entre 3 et 35 Pa.s, mesurée avec un viscosimètre rotatif CONTRAVES TV équipé d'un mobile "MS-r4" à la fréquence de 60 Hz.

La composition selon l'invention étant destinée à une application topique sur la peau ou les phanères, elle contient un milieu physiologiquement acceptable. On entend par "milieu physiologiquement acceptable", un milieu compatible avec la peau, les lèvres, les ongles, le cuir chevelu et/ou les cheveux.

La composition obtenue selon l'invention présente une bonne stabilité dans le temps, même à une température supérieure à la température ambiante (par exemple 45°C).

La composition de l'invention se présente sous la forme d'une crème plus ou moins fluide, c'est-à-dire d'un produit souple par opposition à un produit solide tel qu'un stick. Ainsi, cette composition a une viscosité à la température ambiante (25°C) allant d'environ 10 à 250 poises (1 à 25 Pa.s) et de préférence d'environ 15 à 150 poises (1,5 à 15 Pa.s), cette viscosité étant mesurée avec un Rhéomat 180 à 25°C.

La composition de l'invention, bien que contenant un fort taux de cire, peut contenir un taux important de phase aqueuse. Elle peut ainsi constituer une composition fraîche à l'application. En outre, comme indiqué précédemment, le mélange de la phase aqueuse et de la phase huileuse étant fait à froid, on peut incorporer des composés thermosensibles sans craindre leur dégradation.

### Oligomères et polymères dérivés de polyoléfine

Les oligomères et polymères utilisés dans la composition de l'invention sont constitués d'une partie apolaire polyoléfinique et d'au moins une partie polaire. Ils peuvent présenter une structure de type bloc ou peigne.

La partie apolaire polyoléfinique comprend au moins 40 atomes de carbone et de préférence de 60 à 700 atomes de carbone. Il est important que cette partie comporte au moins 40 atomes de carbone pour atteindre un système bien stable. Cette partie apolaire peut être choisie parmi les polyoléfines telles que les oligomères, les polymères et/ou les copolymères d'éthylène, de propylène, de 1-butène, d'isobutène, de 1-pentène, de 2-méthyl-1-butène, de 3-methyl-1-butène, de 1-héxène, de 1-heptène, de 1-octène, de 1-décène, de 1-undécène, de 1-dodécéne, de 1-tridécène, de 1-tetradécène, de 1-pentadécène, de 1-hexadécéne, de 1-heptadécène et de 1-octadécéne. Ces polyoléfines peuvent être hydrogénées ou non.

La partie polaire des oligomères ou polymères de l'invention peut être anionique, cationique, non ionique, zwitterionique ou amphotère. Elle peut être par exemple constituée de polyalkylène glycols ou de polyalkylène imines, ou encore d'acides ou de diacides carboxyliques, de leurs anhydrides ou de leurs dérivés, et leurs mélanges. Des oligomères ou polymères à partie polaire acide carboxylique peuvent être par exemple issus de la réaction entre une polyoléfine et au moins un acide ou anhydride carboxylique choisi dans le groupe comprenant l'acide succinique, l'anhydride succinique, l'acide maléique, l'anhydride maléique, l'acide fumarique, l'acide itaconique, l'acide citraconique, l'acide mésaconique, l'acide aconitique. De préférence, la partie polaire est constituée par l'acide ou l'anhydride succinique, leurs dérivés notamment esters ou amides, les sels d'ions alcalins, alcalino-terreux ou organiques correspondants, ou bien encore par du polyoxyéthylène.

Les polymères dérivés de polyoxyéthylène peuvent être par exemple choisis parmi les polymères diblocs polyisoprène-polyoxyéthylène, les polymères poly(éthylène-co-propylène)-polyoxyéthylène et leurs mélanges. Ces polymères sont décrits dans la publication de Allgaier, Poppe, Willner, Richter (Macromolecules, 1997, vol. 30, p.1582-1586).

Les polymères dérivés d'acide ou d'anhydride succinique peuvent être choisis notamment parmi les dérivés polyoléfines d'acide ou d'anhydride succinique décrits dans les documents US-A-4,234,435, US-A-4,708,753, US-A-5,129,972, US-A-4,931,110, GB-A-2,156,799 et US-A-4,919,179 incorporés ici pour référence. La partie polyoléfine peut être constituée par exemple de polyisobutylène, hydrogéné ou non, de poids moléculaire allant de 400 à 5000. Dans le polyisobutylène à terminaison succinique ainsi obtenu, la partie succinique peut être estérifiée, amidifiée ou être sous forme de sel, c'est-à-dire qu'elle peut être modifiée par des alcools, des amines, des alcanolamines ou des polyols, ou encore se trouver sous forme de sels de métal alcalin ou alcalino-terreux, d'ammonium ou encore de base organique comme les sels de diéthanolamine et de triéthanolamine. Les polyoléfines à terminaison succinique estérifiée ou amidifiée sont des produits de réaction de (a) une polyoléfine à terminaison succinique, et de (b) une amine ou un alcool, pour former une amide ou un ester. Le terme « amine » utilisé ici comprend tous types d'amines dont les alcanolamines. Il peut s'agir par exemple de mono-amines primaires, secondaires ou tertiaires, ces amines pouvant être aliphatiques, cycloaliphatiques, aromatiques, hétérocycliques, saturées ou insaturées. Par ailleurs, les alcools peuvent être des mono- ou poly-alcools. Les mono-alcools comprennent les alcools aliphatiques primaires, secondaires ou tertiaires, et les phénols. Les poly-alcools peuvent être par exemple choisis parmi les poly-alcools aliphatiques, cycloaliphatiques, aromatiques et hétérocycliques. Les polyoléfines à terminaison succinique modifiée (estérifiée ou amidifiée) et leur procédé de préparation sont décrits en particulier dans le document US-A-4,708,753 qui est incorporé ici pour référence.

Comme polyoléfines à terminaison succinique, on peut citer notamment les polyisobutylènes à terminaison succinique modifiée, tels que les produits commercialisés sous les dénominations LUBRIZOL 2724 et LUBRIZOL 5603 par la société Lubrizol. Selon un mode préféré de réalisation de l'invention, on utilise le polymère commercialisé sous la dénomination LUBRIZOL 5603 par la société Lubrizol, qui est le sel de diéthyléthanolamine de polyisobutylène à terminaison succinique estérifiée (nom INCI : Ethanoldiethonium polyisobutenyl triethylaminosuccinate / diethylethanolamine).

Un autre exemple de polymère utilisable dans l'invention est le produit de la réaction de l'anhydride maléique avec le polyisobutylène, tel que les produits commercialisés sous les dénominations Glissopal (Glissopal 2300, 1300 et 1000) (nom INCI : polyisobutene) par la société BASF.

La quantité d'oligomère(s) ou de polymère(s) en matière active dans la composition de l'invention peut aller par exemple de 0,1 à 10 % en poids, de préférence de 0,5 à 5 % en poids et mieux de 1 à 3 % en poids par rapport au poids total de la composition. On peut utiliser un ou plusieurs oligomères ou polymères dérivés de polyoléfines.

Selon un mode préféré de réalisation de l'invention, la composition selon l'invention est exempte de tout tensioactif habituellement utilisé dans les émulsions E/H, et notamment exempte de tensioactif siliconé.

### Phase huileuse

La composition de l'invention contient dans la phase huileuse, au moins une cire, de préférence au moins 3 % en poids d'une ou plusieurs cires, par rapport au poids total de la composition. La quantité de cire(s) dans la composition de l'invention est de préférence d'au moins 3 %, et elle peut aller par exemple de 3 à 30 %, mieux de 4 à 15 % en poids et encore mieux de 4 à 10 % en poids par rapport au poids total de la composition.

Comme cires utilisables dans la composition de l'invention, on peut citer par exemple les cires minérales telles que les cires microcristallines, la paraffine, la vaseline, l'ozokérite, la cire de montan ; les cires d'origine animale telles que la cire d'abeilles, la lanoline et ses dérivés ; les cires d'origine végétale telles que les cires de Candellila, d'Ouricurry, de Carnauba, du Japon, le beurre de cacao, les cires de fibres de lièges ou de canne à sucre ; les huiles hydrogénées concrètes à 25°C ; les esters gras et les glycérides concrets à 25°C ; les cires synthétiques telles que les cires de polyéthylène et les cires obtenues par synthèse de Fischer-Tropsch ; les cires de silicone, et leurs mélanges.

Selon un mode préféré de réalisation de l'invention, on utilise au moins une cire ayant une température de fusion commençante supérieure ou égale à 50°C, et mieux au moins une cire dont la température de fusion commençante est supérieure à 65°C, telles que la cire de Carnauba, certaines cires de polyéthylène et certaines cires microcristallines telles que celle vendue par la société Tisco sous le nom "Tisco Wax 88" ou celle vendue par la société RMC sous le nom de "Feruwax 30540".

Par. "température de fusion commençante", on entend dans la présente demande, la température à laquelle une cire commence à fondre. On peut déterminer cette température par ATD (analyse thermique différentielle) qui permet l'obtention du thermogramme (ou courbe de fusion) de la cire considérée. La température de fusion commençante correspond à la température à laquelle on peut observer un changement de pente notable dans le thermogramme. Le point de fusion, quant à lui, représente le point minimum dudit thermogramme.

Outre les cires, la phase huileuse de la composition selon l'invention peut contenir au moins un corps gras choisi parmi les huiles liquides à température ambiante (20-25°C), volatiles ou non, les gommes et les corps gras pâteux, d'origine animale, végétale, minérale ou synthétique, et leurs mélanges. Ces corps gras sont physiologiquement acceptables.

Selon un mode préféré de réalisation de l'invention, la phase huileuse contient au moins une huile. On entend par "huile" un corps gras liquide à la température ambiante (25°C).

Comme huiles utilisables dans la composition de l'invention, on peut citer par exemple :
- les huiles hydrocarbonées d'origine animale, telles que le perhydrosqualène (ou squalane) ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de coriandre, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité ;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; les esters du pentaérythritol comme le tétraisostéarate de pentaérythrytyle ; les dérivés lipophiles d'acides aminés, tels que le lauroyl sarcosinate d'isopropyle (nom INCI : Isopropyl Lauroyl Sarcosinate) commercialisé sous la dénomination Eldew SL 205 par la société Ajinomoto ;
- les hydrocarbures linéaires ou ramifiés, d'origine minérale ou synthétique, tels que les huiles minérales (mélange d'huiles hydrocarbonées dérivées du pétrole ; nom INCI : Mineral oil), les huiles de paraffine, volatiles ou non, et leurs dérivés, l'huile de vaseline, les polydécènes, l'isohexadecane, l'isododecane, l'isoparaffine hydrogéné tel que l'huile de Parléam® commercialisée par la société NOF Corporation (nom INCI ; Hydrogenated Polyisobutene) ;
- des alcools gras ayant de 8 à 26 atomes de carbone, comme l'alcool cétylique, l'alcool stéarylique et leur mélange (alcool cétéarylique), l'octyl dodécanol, le 2-butyloctanol, le 2-hexyldécanol, le 2-undécylpentadécanol ou l'alcool oléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclopentasiloxane et la cyclohexadiméthylsiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

Selon un mode préféré de réalisation de l'invention, la composition contient au moins une huile choisie parmi les hydrocarbures linéaires ou ramifiés, d'origine minérale ou synthétique, tels que l'huile de Parléam.

Les autres corps gras pouvant être présents dans la phase huileuse sont par exemple les acides gras comportant de 8 à 30 atomes de carbone, comme l'acide stéarique, l'acide laurique, l'acide palmitique ; les gommes telles que les gommes de silicone (diméthiconol) ; les résines de silicone telles que la trifluorométhyl-C1-4-alkyldimethicone et la Trifluoropropyldimethicone, et les élastomères de silicone comme les produits commercialisés sous les dénominations "KSG" par la société Shin-Etsu, sous la dénomination "Trefil" par la société Dow Corning ou sous les dénominations "Gransil" par la société Grant Industries ; les pâtes telles que Petrolatum ; et leurs mélanges.

Selon un mode particulier de réalisation de l'invention, la composition contient au moins une gomme de silicone dont la présence améliore encore les qualités cosmétiques de la composition. On utilise de préférence une ou plusieurs gommes de silicone ayant un poids moléculaire inférieur à 1 500 000, telle qu'un polydiméthylsiloxane (nom INCI : dimethicone), un polyphénylsiloxane ou un polyhydroxysiloxane (nom INCI : dimethiconol). Quand elle est présente, la quantité de gomme de silicone peut aller par exemple de 0,01 à 10 % en poids, de préférence de 0,1 à 3 % en poids par rapport au poids de la composition finale.

La phase huileuse comprenant tous les corps gras et les adjuvants lipidiques éventuellement présents (par exemple charges, actifs, etc) est présente dans la composition selon l'invention en une quantité allant généralement de 5 à 70 %, de préférence de 10 à 50 % en poids et mieux de 10 à 30 % en poids par rapport au poids total de la composition.

Comme indiqué ci-dessus, la phase huileuse refroidie se présente de façon avantageuse, avant son mélange avec la phase aqueuse, sous forme d'une pâte souple, c'est-à-dire ayant une viscosité dynamique de la pâte souple à 25°C est généralement comprise entre 3 et 35 Pa.s, mesurée avec un viscosimètre rotatif CONTRAVES TV équipé d'un mobile "MS-r4" à la fréquence de 60 Hz.

Selon un mode particulier de réalisation de l'invention, on réalise une pâte anhydre souple à partir des cires, de tout ou d'une partie de l'huile (généralement un hydrocarbure linéaire ou ramifié, d'origine minérale ou synthétique, telle qu'une huile minérale ou l'huile de Parleam), et éventuellement des charges, puis on ajoute à la pâte obtenue, les autres huiles et constituants de la phase huileuse, la phase huileuse finale obtenue étant elle-même sous forme d'une pâte souple.

### Phase aqueuse

La phase aqueuse de la composition de l'invention peut aller de 95 à 30 % en poids, de préférence de 90 à 50 % en poids et mieux de 90 à 70 % en poids par rapport au poids total de la composition. Elle contient au moins de l'eau. Elle peut contenir, outre l'eau, un ou plusieurs composés miscibles à l'eau ou au moins en partie miscibles à l'eau, comme les polyols ; les mono-alcools inférieurs en C₂ à C₈, tels que l'éthanol et l'isopropanol ; et les cétones en C₃ à C₄ liquides à température ambiante. Par "température ambiante", il faut comprendre une température d'environ 25°C, à pression atmosphérique normale (760 mm de Hg).

Par "polyol", il faut comprendre toute molécule organique comportant au moins deux groupements hydroxyle libres. Comme polyols, on peut citer par exemple la glycérine, les glycols comme le butylène glycol, le propylène glycol, l'isoprène glycol, le dipropylene glycol, le pentylene glycol, l'hexylene glycol et les polyéthylène glycols comme le PEG-8, le sorbitol, les sucres comme le glucose.

Le ou les solvants peuvent être présents en une quantité allant de 0,1 à 30 % en poids par rapport au poids total de la composition.

### Adjuvants

De façon connue, la composition de l'invention peut contenir également des adjuvants habituels dans les domaines cosmétique et/ou dermatologique, tels que les actifs, les conservateurs, les antioxydants, les agents complexants, les ajusteurs de pH (acides ou basiques), les parfums, les charges, les bactéricides, les absorbeurs d'odeur, les matières colorantes (pigments et colorants) et encore les vésicules lipidiques. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse, dans la phase aqueuse et/ou dans les vésicules lipidiques.

Selon un mode préféré de réalisation de l'invention, la composition contient une ou plusieurs charges.

Les charges peuvent être choisies notamment parmi les poudres (organiques ou minérales) et les fibres.

Comme poudres qui peuvent être utilisées dans la composition de l'invention, on peut citer par exemple, les oxydes de zinc ; les oxydes de titane tels que le dioxyde de titane ; les micas d'origine naturelle ou synthétique ; le carbonate de calcium ; le carbonate et l'hydrocarbonate de magnésium ; la silice, en particulier la silice sphérique, la poudre de silice commercialisée sous la dénomination Cab-O-Sil TS 530 par la société Cabot, et les microbilles de silice telles que celles commercialisées sous la dénomination SB150 par la société Myoshi ; le talc ; le kaolin ; les billes de verre et de céramique commercialisées par la société 3M sous la dénomination commerciale MACROLITE ; les savons métalliques dérivés d'acide organique carboxylique ayant de 8 à 22 atomes de carbone, de préférence de 12 à 18 atomes de carbone, par exemple le stéarate de zinc, de magnésium ou de lithium, le laurate de zinc, le myristate de magnésium ; les particules de polyamide et notamment celles vendues sous les dénominations ORGASOL par la société Atochem ; les poudres de polymères synthétiques non expansées, telles que les poudres de polyéthylène, de polystyrène, de polyesters, de polyamides (par exemple le nylon ou la poly-β-alanine), de copolymères d'acrylates (par exemple les microsphères microporeuses vendues par la société Dow Corning sous la dénomination commerciale POLYTRAP), d'acides polyméthacryliques, de Téflon® (polytétrafluuoroéthylène) comme les produits commercialisés sous les dénominations FLUON par la société Uniqema ; les poudres expansées telles que les microsphères creuses en matériau thermoplastique préparées par des procédés connus, comme ceux décrits dans les documents US-A-3 615 972 et EP-A-0 56219, et notamment, les microsphères commercialisées sous les dénominations EXPANCEL par la société Kemanord Plast ou sous la dénomination MICROPEARL F 80 ED par la société Matsumoto ; les poudres de matériaux organiques naturels tels que les amidons de maïs, de blé ou de riz, réticulés ou non, telles que les poudres d'amidon réticulé par l'anhydride octénylsuccinate, commercialisées sous la dénomination DRY-FLO par la société National Starch ; les microbilles de résine de silicone telles que celles commercialisées sous la dénomination TOSPEARL par la société Toshiba Silicone ; et leurs mélanges.

Les fibres éventuellement présentes dans la composition de l'invention sont choisies de préférence parmi les fibres de soie, de coton, de laine, de lin, de cellulose extraites notamment du bois, des légumes ou des algues, de polyamide, de rayonne, de viscose, d'acétate notamment d'acétate de rayonne, d'acétate de cellulose ou d'acétate de soie, de poly-p-phénylène téréphtamide, en acrylique notamment de polyméthacrylate de méthyle ou de poly-2-hydroxyéthylméthacrylate, de polyoléfine et notamment de polyéthylène ou de polypropylène, de verre, de silice, d'aramide, de carbone notamment sous forme graphite, de Téflon®, de collagène insoluble, de polyesters, de polychlorure de vinyle ou de vinylidène, d'alcool polyvinylique, de polyacrylonitrile, de chitosane, de polyuréthane, de polyéthylène phtalate, des fibres de mélanges de polymères, des fibres chirurgicales, et leurs mélanges.

Comme fibres, on utilise préférentiellement, les fibres de polyamide, notamment en Nylon®, en particulier celles commercialisées sous les appellations Nylon 6 = Polyamide 6 ; Nylon 66 = Polyamide 6,6 ; Nylon 12 = Polyamide 12).

Quand elles sont présentes, ces charges peuvent être en des quantités allant par exemple de 0,1 à 20 % en poids, de préférence de 0,5 à 15 % en poids et mieux de 0,5 à 10 % en poids par rapport au poids total de la composition.

Comme actifs utilisables dans la composition de l'invention, on peut citer par exemple, les flavonoïdes ; les agents hydratants tels que les hydrolysats de protéines ; le hyaluronate de sodium ; les polyols comme la glycérine, les glycols comme les polyéthylène glycols, et les dérivés de sucre ; les anti-inflammatoires ; les oligomères procyannidoliques ; les vitamines comme la vitamine A (rétinol), la vitamine E (tocophérol), les dérivés de ces vitamines (notamment esters) et leurs mélanges ; l'urée ; la caféine ; les dépigmentants tels que l'hydroquinone et l'acide caféique ; l'acide salicylique et ses dérivés ; les alpha-hydroxyacides tels que l'acide lactique et l'acide glycolique et leurs dérivés ; les rétinoïdes tels que les caroténoïdes et les dérivés de vitamine A; les filtres solaires ; l'hydrocortisone ; la mélatonine ; les extraits d'algues, de champignons, de végétaux, de levures, de bactéries ; les stéroïdes ; les actifs anti-bactériens comme le 2,4,4'-trichloro-2'-hydroxy diphényl éther (ou triclosan), le 3,4,4'-trichlorocarbanilide (ou triclocarban) et les acides indiqués ci-dessus et notamment l'acide salicylique et ses dérivés ; les agents matifiants ; les agents tenseurs ; les céramides ; les huiles essentielles ; et leurs mélanges ; et tout actif approprié pour le but final de la composition.

Comme exemples de stéroïdes, on peut citer la déhydroépiandrostérone (ou DHEA), ainsi que (1) ses précurseurs et dérivés biologiques, en particulier les sels et esters de DHEA, tels que le sulfate et le salicylate de DHEA, la 7-hydroxy DHEA, la 7-céto DHEA, les esters de 7-hydroxy et 7-céto DHEA, notamment la 3-beta-acétoxy-7-oxo DHEA, et (2) ses précurseurs et dérivés chimiques, en particulier les sapogénines telles que la diosgénine ou l'hécogénine, et/ou leurs dérivés tels que l'acétate d'hécogénine, et/ou les extraits naturels en contenant et notamment les extraits de Dioscorées, tels que l'igname sauvage (Wild Yam).

Les filtres solaires peuvent être choisis parmi les filtres UV organiques, tels que les composés suivants :
- Les dérivés salicyliques et notamment l'éthylhexyl salicylate (ou salicylate d'éthyl hexyle) vendu sous le nom commercial NEO HELIOPAN OS par HAARMANN et REIMER ;
- Les dérivés du dibenzoylméthane et notamment le Butyl méthoxydibenzoylmethane vendu notamment sous le nom commercial PARSOL 1789 par HOFFMANN LA ROCHE ;
- Les dérivés cinnamiques et notamment l'éthylhexyl méthoxycinnamate vendu notamment sous le nom commercial PARSOL MCX par HOFFMANN LA ROCHE ;
- Les dérivés de β,β'-diphénylacrylate et notamment l'octocrylène (α-cyano-β,β-diphénylacrylate de 2-éthylhexyle) vendu notamment sous le nom commercial UVINUL N539 par BASF ;
- L'acide phenylbenzimidazole sulfonique ;
- Les dérivés du benzylidène camphre, et notamment l'acide téréphthalylidène dicamphosulfonique (Terephthalylidene Dicamphor Sulfonic) fabriqué sous le nom MEXORYL SX par CHIMEX, et le 4-méthylbenzylidène camphre vendu sous le nom commercial EUSOLEX 6300 par MERCK ;
- Les dérivés de la benzophénone, et notamment la Benzophenone-3 ou Oxybenzone, vendue sous le nom commercial UVINUL M40 par BASF ; la Benzophenone-4 vendue sous le nom commercial UVINUL MS40 par BASF ; la Benzophenone-5 ;
- Les dérivés du phényl benzimidazole, et notamment le Benzimidazilate vendu sous le nom commercial commercial NEO HELIOPAN AP par HAARMANN et REIMER ;
- Les dérivés de la triazine, et notamment l'anisotriazine vendu sous le nom commercial TINOSORB S par CIBA GEIGY ; l'éthylhexyl triazone vendu notamment sous le nom commercial UVINUL T150 par BASF ; et le diéthylhexyl Butamido Triazone vendu sous le nom commercial UVASORB HEB par SIGMA 3V ;
- Le méthylène bis-benzotriazolyl tétraméthylbutylphenol ;
- Les dérivés du phényl benzotriazole, et notamment le Drometrizole Trisiloxane vendu sous le nom commercial SILATRIZOLE par RHODIA CHIMIE ;
- et leurs mélanges.

Les filtres solaires peuvent être choisis aussi parmi les filtres physiques. Comme filtres physiques, on peut citer par exemple les pigments et nano-pigments d'oxydes métalliques, enrobés ou non enrobés, notamment les oxydes de titane, de fer, de zirconium, de zinc ou de cérium, et leurs mélanges, ces oxydes pouvant être sous forme de micro- ou nanoparticules (nanopigments), éventuellement enrobées.

La quantité d'actifs dépend du but recherché. Le ou les actifs peuvent être par exemple présents en une concentration allant de 0,001 à 20 %, de préférence de 0,01 à 10 % en poids et mieux de 0,05 à 5 % du poids total de la composition.

Par ailleurs, la composition selon l'invention peut éventuellement contenir un ou plusieurs polymères autres que les oligomères ou polymères dérivés de polyoléfine. On peut citer comme polymères utilisables dans la composition de l'invention : les polymères carboxyvinyliques modifiés ou non, tels que les produits commercialisés sous les dénominations Carbopol (nom INCI : carbomer) et Pemulen (nom INCI : Acrylates/C10-30 akyl acrylate crosspolymer) par la société Goodrich ; les polyacrylamides ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société Hoechst sous la dénomination « Hostacerin AMPS » (nom INCI : ammonium polyacryldimethyltaurate) ; les copolymères anioniques réticulés d'acrylamide et d'AMPS, se présentant sous la forme d'une émulsion, tels ceux commercialisés sous le nom de SEPIGEL 305 (nom C.T.F.A. : Polyacrylamide / C13-14 Isoparaffin / Laureth-7) et sous le nom de SIMULGEL 600 (nom C.T.F.A. : Acrylamide / Sodium acryloyldimethyltaurate copolymer /

Isohexadecane / Polysorbate 80) par la société SEPPIC ; les copolymères acrylate/acrylonitrile tels que le HYPAN SS201 commercialisé par la société Kingston ; les biopolymères polysaccharidiques comme la gomme de xanthane, la gomme de guar, les alginates, les celluloses modifiées ou non ; les composés inorganiques tels que les smectites, les hectorites modifiées ou non telles que les produits BENTONE commercialisés par la société Rheox, les produits LAPONITE commercialisés par la société Southern Clay Products, le produit VEEGUM HS commercialisé par la société R.T.Vanderbilt ; et leurs mélanges.

Quand ils sont présents, la quantité de ces polymères peut aller par exemple de 0,05 à 2 % en poids par rapport au poids total de la composition.

Bien entendu, l'homme de l'art veillera à choisir le ou les éventuels composés à ajouter à la composition selon l'invention et leurs quantités, de manière telle que les propriétés avantageuses attachées intrinsèquement à la composition conforme à l'invention ne soient pas ou substantiellement pas, altérées par l'addition envisagée.

La composition selon l'invention peut se présenter notamment sous forme d'une crème plus ou moins fluide, et elle peut constituer notamment une composition cosmétique ou dermatologique. Elle trouve alors son application dans un grand nombre de traitements notamment cosmétiques de la peau, y compris du cuir chevelu, des cheveux, des ongles, et/ou des muqueuses, en particulier pour le soin, la protection et/ou le maquillage de la peau du corps ou du visage, des cils et/ou des lèvres, et/ou pour le soin des cheveux (par exemple masque pour cheveux).

Aussi, la présente invention a pour objet l'utilisation cosmétique d'une composition cosmétique telle que définie ci-dessus, pour le soin, la protection et/ou pour le maquillage de la peau et/ou des lèvres, et/ou pour le soin des cheveux.

La présente invention a encore pour objet un procédé de traitement cosmétique de la peau, y compris du cuir chevelu, des cheveux, et/ou des lèvres, caractérisé par le fait que l'on applique sur la peau, les cheveux et/ou les lèvres, une composition cosmétique telle que définie ci-dessus.

Par ailleurs, la composition selon l'invention est bien tolérée par les peaux sensibles. Ainsi, la présente invention a encore pour objet l'utilisation cosmétique d'une composition cosmétique telle que définie ci-dessus, pour le soin, la protection et/ou pour le maquillage des peaux sensibles.

La composition selon l'invention peut être préparée de manière avantageuse en utilisant, pour au moins une étape du procédé, un appareil de malaxage tel qu'un broyeur à cylindres comportant deux cylindres tournant en sens inverse entre lesquels passe la pâte, ou tel qu'un mélangeur-extrudeur à vis. On peut réaliser une ou plusieurs étapes de la préparation de l'émulsion ou toutes les étapes de préparation dans un tel appareil.

Pour incorporer de manière efficace les cires et les charges éventuellement présentes, il est avantageux de réaliser la préparation de la composition ou au moins une étape de cette préparation, en particulier la préparation de la phase huileuse ou une étape de préparation de la phase huileuse, dans un mélangeur-extrudeur à vis, soumis à un gradient de température allant de 100°C à 20°C. Il est en particulier avantageux de réaliser le mélange des charges, des cires et d'au moins une huile qui peut être notamment un hydrocarbure linéaire ou ramifié, dans un mélangeur-extrudeur à vis, soumis à un gradient de température allant de 100°C à 20°C.

Un autre objet de l'invention est donc un procédé de préparation d'une composition selon l'invention, caractérisé en ce que l'on effectue au moins une étape dans un mélangeur-extrudeur à vis, soumis à un gradient de température allant de 100°C à 20°C.

Le procédé de préparation peut être exécuté de différentes manières. Selon un mode préféré de réalisation de l'invention, le procédé de préparation peut comprendre les étapes suivantes :
(1) introduction de la ou des cires, d'au moins une huile, et d'additifs éventuels (par exemple charges, gommes) dans le fourreau chauffé à une température allant d'environ 80°C à environ 100°C, d'un mélangeur-extrudeur comportant plusieurs fourreaux portés à des températures décroissantes allant de 100°C à 20°C ;
(2) malaxage du mélange dans le mélangeur-extrudeur, pour obtenir à la sortie du mélangeur-extrudeur, une pâte anhydre froide souple ;
(3) mélange de la pâte obtenue avec le reste de la phase huileuse sous agitation, et obtention d'une pâte souple à température ambiante (20°C) ;
(4) préparation de la phase aqueuse ;
(5) introduction de la phase aqueuse dans la phase huileuse, puis addition éventuelle des additifs devant être ajoutés après émulsification (certaines charges par exemple).

Selon une variante de ce procédé, l'étape (3) ou toutes les étapes peuvent être réalisées dans un mélangeur-extrudeur faisant suite au premier. Ainsi un autre mode opératoire peut consister :
- chauffer le mélange de cire et d'huile à environ 100°C ;
- à introduire les charges dans le premier fourreau d'un mélangeur-extrudeur comportant six fourreaux portés à des températures décroissantes allant de 100°C à 20°C ;
- à introduire le mélange fondu de cire et huile dans le second élément dudit mélangeur-extrudeur à vis, et
- à introduire la phase aqueuse et l'émulsionnant polymérique par deux entrées différentes, dans le quatrième élément dudit mélangeur-extrudeur à vis.

Les éléments du mélangeur-extrudeur bi vis utilisé sont, en allant du premier au sixième élément, par exemple portés respectivement aux températures suivantes : 100°C, 80°C, 60°C, 20°C, 20°C et 20°C.

L'utilisation d'un mélangeur-extrudeur permet d'obtenir de façon reproductible une pâte de phase huileuse de qualité très constante. De plus, il est possible, en adaptant la filière de sortie du mélangeur-extrudeur, de conditionner la composition en ligne à la sortie dudit mélangeur-extrudeur.

Les différents étapes du procédé peuvent être réalisées dans un ou plusieurs extrudeurs disposés à la suite les uns des autres, et de préférence dans un extrudeur bivis unique.

Les conditions dans lesquelles l'extrusion peut être effectuée sont décrites dans le document FR-A-2,715,306 dont le contenu est incorporé à la présente demande par référence.

La longueur des fourreaux peut varier. De manière préférée, elle est de 100 mm, et on peut ajouter des fourreaux supplémentaires si nécessaire pour l'introduction séparée d'autres composés.

L'exemple ci-après de compositions selon l'invention est donné à titre d'illustration et sans caractère limitatif. Les quantités y sont données en % en poids, sauf mention contraire.

### Exemple 1 selon l'invention

### Pâte anhydre

- Fibres (Nylon 66) 19 %
- Silice 5 %
- Gomme de silicone 4 %
- Cire microcristalline 19 %
- Huile de Parleam qsp 100 %

### Emulsion E/H

### Phase A

- Pâte anhydre 15 %
- Lubrizol 5603 1,92 %
- Isononanoate d'isononyle 4,65 %
- Squalane 3,48 %
- diméthicone 2,33 %

### Phase B

- Glycérine 5 %
- sel (Sulfate de magnésium) 2 %
- Ammonium polyacryloyldiméthyltaurate (Hostacerin AMPS) 0,2 %
- conservateur 1 %
- Eau qsp 100 %

### Phase C

- Amidon (Dry Flo) 3 %

### Mode opératoire :

1) on prépare la pâte anhydre en introduisant dans le fourreau chauffé à 100°C d'un mélangeur-extrudeur, les charges (fibres, silice) et la gomme de silicone et en y ajoutant le mélange de cire et d'huile de Parleam. On obtient à la sortie du mélangeur-extrudeur, une pâte souple.
2) on prépare la phase A (phase huileuse) en faisant ramollir le Lubrizol 5603 à 80°C pendant 10-15 minutes, on y ajoutant la pâte souple puis le reste de la phase A sous agitation.
3) on prépare la phase B (phase aqueuse) en dispersant l'Hostacerin AMPS dans les autres constituants sous agitation pendant 10-15 minutes.
4) on incorpore lentement la phase B dans la phase A sous agitation, puis on ajoute la phase C au mélange sous agitation.

L'émulsion E/H obtenue a une viscosité de 24,9 poises (2,49 Pa.s). Elle est fluide et filmogène, et, au microscope, elle est fine et régulière. Elle reste stable et fine après un mois en cycles de températures à 4°C, à 25°C et à 45°C. Elle est apte à être utilisée comme crème de soin tout en apportant beaucoup de douceur à la peau et étant bien tolérée par les peaux sensibles.

### Exemple 2 selon l'invention

### Pâte anhydre

- Fibres (Nylon 66) 19 %
- Gomme de silicone 4 %
- Cire microcristalline 19 %
- Huile de Parleam qsp 100 %

### Emulsion E/H

### Phase A

- Pâte anhydre 7,5 %
- Lubrizol 5603 1,92 %
- Isononanoate d'isononyle 2,32 %
- Squalane 1,74 %
- diméthicone 1,17 %

### Phase B

- Glycérine 5 %
- sel (sulfate de magnésium) 2 %
- Ammonium polyacryloyldiméthyltaurate (Hostacerin AMPS) 0,2 %
- conservateur 1 %
- Eau qsp 100 %

### Phase C

- Amidon (Dry Flo) 3 %

Mode opératoire : identique à celui de l'exemple 1.

L'émulsion E/H obtenue a une viscosité de 95 poises (9,5 Pa.s). Elle a l'aspect d'une crème et elle est macroscopiquement homogène. De plus, au microscope, elle est fine et régulière. Elle reste stable et fine après un mois en cycles de températures à 4°C, à 25°C et à 45°C. Elle est apte à être utilisée comme crème de soin tout en apportant beaucoup de douceur à la peau et étant bien tolérée par les peaux sensibles.

### Exemple 3 comparatif

### Pâte anhydre

- Fibres (Nylon 66) 19 %
- Gomme de silicone 4 %
- Cire microcristalline 19 %
- Huile de Parleam qsp 100 %

### Emulsion H/E

### Phase A

- Pâte anhydre 5 %
- Cyclopentasiloxane 7,5 %
- Huile végétale 10 %

### Phase B

- Glycérine 7 %
- Copolymère de phtalate / sulfoisophtalate / glycol (AQ38S) 3,4 %
- conservateur 1 %
- Eau qsp 100 %

### Phase C

- Ammonium polyacryloyldiméthyltaurate (Hostacerin AMPS) 0,2 %

Le mode opératoire consiste à préparer la pâte anhydre comme décrit dans l'exemple 1, à préparer la phase huileuse (A) et à homogénéiser la phase B, puis à disperser la phase C dans la phase B, et à verser le mélange des phases B et C dans la phase huileuse A sous agitation.

L'émulsion E/H obtenue est instable après 24 heures : il se produit une déphasage avec une remontée d'huile sur le dessus de l'émulsion.

## Revendications

1. Composition constituée d'une émulsion E/H comprenant, dans un milieu physiologiquement acceptable, une phase aqueuse dispersée dans une phase huileuse, la phase huileuse contenant au moins une cire et se présentant sous forme d'une pâte souple à température ambiante, **caractérisée en ce qu'**elle contient au moins un polymère amphiphile choisi parmi les oligomères ou polymères dérivés de polyoléfine, comportant une partie apolaire polyoléfinique comprenant au moins 40 atomes de carbone et au moins une partie polaire.

2. Composition selon la revendication précédente, **caractérisée en ce que** l'oligomère ou le polymère dérivé de polyoléfine est un polyisobutylène à terminaison succinique modifiée.

3. Composition selon la revendication 1 ou 2 , **caractérisée en ce que** l'oligomère ou le polymère dérivé de polyoléfine est le produit de la réaction de l'anhydride maléique avec le polyisobutylène.

4. Composition selon l'une quelconque des revendications précédentes, **caractérisée en ce que** la quantité d'oligomère ou de polymère dérivé de polyoléfine va de 0,1 à 10 % en poids par rapport au poids total de l'émulsion.

5. Composition selon l'une quelconque des revendications précédentes, **caractérisée en ce que** la cire est choisie dans le groupe comprenant les cires minérales, les cires d'origine animale, les cires d'origine végétale, les huiles hydrogénées concrètes à 25°C, les esters gras et les glycérides concrets à 25°C, les cires synthétiques, les cires de silicone et leurs mélanges.

6. Composition selon l'une quelconque des revendications précédentes, **caractérisée en ce qu'**elle comprend au moins une cire choisie parmi la cire de Carnauba, les cires de polyéthylène ayant une température de fusion commençante supérieure à 65°C, les cires microcristallines ayant une température de fusion commençante supérieure à 65°C, et leurs mélanges.

7. Composition selon l'une quelconque des revendications précédentes, **caractérisée en ce que** la quantité de cire(s) va de 3 à 30 % en poids par rapport au poids total de la composition.

8. Composition selon l'une quelconque des revendications précédentes, **caractérisée par le fait qu'**elle constitue une composition cosmétique ou dermatologique.

9. Utilisation cosmétique d'une composition cosmétique selon l'une quelconque des revendications 1 à 7, pour le soin, la protection et/ou le maquillage de la peau et/ou des lèvres et/ou pour le soin des cheveux.

10. Procédé de traitement cosmétique de la peau, y compris du cuir chevelu, des cheveux, et/ou des lèvres, **caractérisé par le fait que** l'on applique sur la peau, les cheveux et/ou les lèvres, une composition cosmétique selon l'une quelconque des revendications 1 à 7.

11. Procédé de préparation d'une composition selon l'une quelconque des revendications 1 à 8, **caractérisé en ce que** l'on effectue **caractérisé en ce que** l'on effectue au moins une étape dans un mélangeur-extrudeur à vis, soumis à un gradient de température allant de 100°C à 20°C.
